# EUROPEAN PATENT APPLICATION

(11) **EP 4 234 671 A1**
(43) Date of publication of application: **30.08.2023**
(21) Application number: 23157502.8
(22) Date of filing: 20.02.2023
(51) Int. Cl.: C11D 3/386, C12N 9/20

(54) **COMPOSITIONS CONTAINING BIOSURFACTANTS AND A LIPASE FROM STACHYBOTRYS CHLOROHALONATA**

(30) Priority: 24.02.2022 EP 22158413
(71) Applicant: Evonik Operations GmbH, 45128 Essen (DE)
(72) Inventor: Trambitas, Alexandra, 63755 Alzenau (DE); Vashisht, Hitesh, 60385 Frankfurt (DE); Kleinen, Jochen, 52525 Heinsberg (DE); Liebig, Stefan Julian, 40627 Düsseldorf (DE); Pfefferle, Walter, 35428 Langgöns (DE); Schmidt, Harald, 45481 Mülheim an der Ruhr (DE)
(74) Representative: Evonik Patent Association

(57) **Abstract**

Field of the invention are compositions comprising
A) at least one lipase selected from Seq.-ID-No. 1 and its respective at least 60%, preferably at least 80%, more preferably at least 90%, especially preferably at least 95%, 98% or 99%, homologues at the amino acid level,
B) at least one biosurfactant, and optionally
C) at least one non-bio surfactant,
and their use.

## Description

### Field of the invention

Field of the invention are compositions comprising
A) at least one lipase selected from Seq.-ID-No. 1 and its respective at least 60%, preferably at least 80%, more preferably at least 90%, especially preferably at least 95%, 98% or 99%, homologues at the amino acid level,
B) at least one biosurfactant, and optionally
C) at least one non-bio surfactant,
and their use.

### Prior art

EP2596089 discloses a cleaning composition comprising an effective amount of surfactant system and an enzyme system, characterised in that the surfactant system comprises at least 1 wt.-% (based on the cleaning composition) of a biosurfactant, which is a glycolipid surfactant, comprising at least 20 mol% of glycolipid having both disaccharide and acid moieties and at least one lipase enzyme of bacterial origin.

Accession numbers KFA62895.1 of the NCBI protein bank database discloses a hypothetical protein S40285_02264 of *Stachybotrys chlorohalonata* IBT 40285.

It was an objection of the invention to provide for compositions with outstanding properties in the field of stain removal.

### Description of the invention

Surprisingly, it has been found that the compositions described below are able to solve the problem addressed by the invention.

The present invention therefore relates to composition comprising
A) at least one lipase selected from Seq.-ID-No. 1 and its respective at least 60%, preferably at least 80%, more preferably at least 90%, especially preferably at least 95%, 98% or 99%, homologues at the amino acid level,
B) at least one biosurfactant, and optionally
C) at least one non-bio surfactant.

The invention further provides for the use of the inventive compositions to remove fat and/or oil-containing stains.

An advantage of the composition according to the instant invention is that the proportion of surfactants present therein, based on renewable raw materials, is preferably more than 50% by weight, based on the total amount of surfactants present in the composition.

A further advantage of the composition according to the instant invention is that sugars or sugars and glycerides and/or fatty acids can be used as raw materials for the biosurfactants.

A further advantage of the composition according to the instant invention is that it is very mild. Another advantage of the present invention is that, in the compositions according to the invention, the amount of protease inhibitor required may be reduced or the protease inhibitor may even be completely dispensed with.

A further advantage compared to the prior art is the increased stability of the enzymes during the washing process, and the improved cleaning performance linked thereto, for example, in laundry. Another advantage of the present invention is that, in the compositions according to the invention, proteases may also be used for which the stability in detergent formulations has hitherto been insufficient.

Another advantage of the present invention is that no or fewer complexing agents (builders) have to be used to ensure adequate washing performance in hard water.

The instant invention encompasses a composition comprising
A) at least one lipase selected from Seq.-ID-No. 1 and its respective at least 60%, preferably at least 80%, more preferably at least 90%, especially preferably at least 95%, 98% or 99%, homologues at the amino acid level,
B) at least one biosurfactant, and optionally
C) at least one non-bio surfactant.

"Homology at the amino acid level" in the context of the present invention shall be understood here and hereinafter to mean "amino acid identity", which can be determined with the aid of known methods. In general, use is made of special computer programs with algorithms taking into account specific requirements. Preferred methods for determining the identity initially generate the greatest alignment between the sequences to be compared. Computer programs for determining the identity include, but are not limited to, the GCG program package including
- GAP (Deveroy, J. et al., Nucleic Acid Research 12 (1984), page 387, Genetics Computer Group University of Wisconsin, Medicine (WI), and
- BLASTP, BLASTN and FASTA (Altschul, S. et al., Journal of Molecular Biology 215 (1990), pages 403-410. The BLAST program can be obtained from the National Center For Biotechnology Information (NCBI) and from other sources (BLAST Handbook, Altschul S. et al., NCBI NLM NIH Bethesda ND 22894; Altschul S. et al., above).

The person skilled in the art is aware that various computer programs are available for the calculation of similarity or identity between two nucleotide or amino acid sequences. For instance, the percentage identity between two amino acid sequences can be determined, for example, by the algorithm developed by Needleman and Wunsch (J. Mol. Biol. (48): 444-453 (1970)), which has been integrated into the GAP program in the GCG software package (available at http://www.gcg.com), using either a Blossom 62 matrix or a PAM250 matrix, a gap weight of 16, 14, 12, 10, 8, 6 or 4 and a length weight of 1, 2, 3, 4, 5 or 6. The person skilled in the art will recognize that the use of different parameters will lead to slightly different results, but that the percentage identity between two amino acid sequences overall will not be significantly different. Typically, the Blossom 62 matrix is utilized with employment of the default settings (gap weight: 12, length weight: 1).

In the context of the present invention, an identity of 60% according to the above algorithm means 60% homology. The same applies to higher identities.

The accession numbers cited in the context of the present invention correspond to the NCBI protein bank database entries as at 01.01.2020; generally, in the present context, the version number of the entry is identified by ".number", for example "1".

The lipases that are homologous at the amino acid level, by comparison with the reference sequence, preferably have at least 50%, especially at least 90%, enzyme activity in 4-nitrophenyl octanoate Units. One 4-nitrophenyl octanoate Unit is defined as the amount of enzyme that cleaves one µmοl of 4-Nitrophenol per minute from 4-nitrophenyl octanoate at 25°C at a pH of 8,5.

Within the context of the present invention, biosurfactants are understood as meaning all glycolipids produced by fermentation.

Raw materials for producing the biosurfactants that can be used are carbohydrates, in particular sugars such as e.g. glucose and/or lipophilic carbon sources such as fats, oils, partial glycerides, fatty acids, fatty alcohols, long-chain saturated or unsaturated hydrocarbons.

Where average values are stated hereinbelow, then, unless stated otherwise, these are number-averaged average values. Unless stated otherwise, percentages are data in per cent by weight. Wherever measurement values are stated hereinbelow, then, unless stated otherwise, these have been determined at a temperature of 25°C and a pressure of 1013 mbar.

The "pH" in connection with the present invention - unless stated otherwise - is defined as the value which is measured for the relevant composition at 25°C after stirring for five minutes using a pH electrode calibrated in accordance with ISO 4316 (1977).

The composition according to the instant invention preferably comprises as component B) at least one biosurfactant rhamnolipids, sophorolipids, glucolipids, cellulose lipids, mannosylerythritol lipids and/or trehalose lipids, preferably rhamnolipids, glucolipids and/or sophorolipids, most preferably sophorolipids. The biosurfactants, in particular glycolipid surfactants, can be produced e.g. as in EP 0 499 434, US 7,985,722, WO 03/006146, JP 60 183032, DE 19648439, DE 19600743, JP 01 304034, CN 1337439, JP 2006 274233, KR 2004033376, JP 2006 083238, JP 2006 070231, WO 03/002700, FR 2740779, DE 2939519, US 7,556,654, FR 2855752, EP 1445302, JP 2008 062179 and JP 2007 181789 or the documents cited therein. Suitable biosurfactants can be acquired e.g. from Soliance, France.

Preferably, the composition according to the instant invention has, as biosurfactants, rhamnolipids, in particular mono-, di- or polyrhamnolipids, glucolipids and/or sophorolipids, most preferably sophorolipids.

The term "rhamnolipids" in the context of the present invention preferably is understood to mean particularly compounds of the general formula (I) and salts thereof, 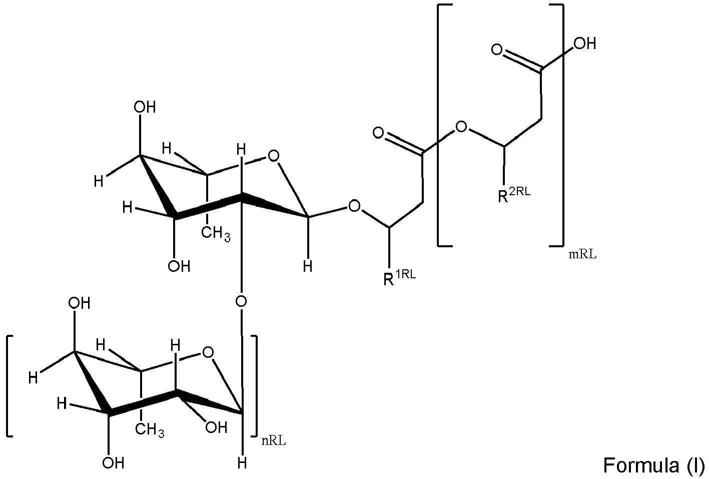 where
mRL = 2, 1 or 0,
nRL = 1 or 0,
R^{1RL} and R^{2RL} = mutually independently, identical or different, organic residues having 2 to 24, preferably 5 to 13 carbon atoms, in particular optionally branched, optionally substituted, particularly hydroxy-substituted, optionally unsaturated, in particular optionally mono-, bi- or triunsaturated alkyl residues, preferably those selected from the group consisting of pentenyl, heptenyl, nonenyl, undecenyl and tridecenyl and (CH₂)ₒ-CH₃ where o = 1 to 23, preferably 4 to 12. If nRL = 1, the glycosidic bond between the two rhamnose units is preferably in the α-configuration. The optically active carbon atoms of the fatty acids are preferably present as R-enantiomers (e.g. (R)-3-{(R)-3-[2-O-(α-L-rhamnopyranosyl)-α-L-rhamnopyranosyl]oxydecanoyl}oxydecanoate).

The term "di-rhamnolipid" in the context of the present invention is understood to mean compounds of the general formula (I) or salts thereof, where nRL = 1.

The term "mono-rhamnolipid" in the context of the present invention is understood to mean compounds of the general formula (I) or salts thereof, where nRL = 0.

Distinct rhamnolipids are abbreviated according to the following nomenclature:
"diRL-CXCY" are understood to mean di-rhamnolipids of the general formula (I), in which one of the residues R^{1RL} and R^{2RL} = (CH₂)ₒ-CH₃ where o = X-4 and the remaining residue R¹ or R² = (CH₂)ₒ-CH₃ where o = Y-4.
"monoRL-CXCY" are understood to mean mono-rhamnolipids of the general formula (I), in which one of the residues R^{1RL} and R^{2RL} = (CH₂)ₒ-CH₃ where o = X-4 and the remaining residue R^{1RL} or R^{2RL} = (CH₂)ₒ-CH₃ where o = Y-4.

The nomenclature used therefore does not distinguish between "CXCY" and "CYCX".

For rhamnolipids where mRL=0, monoRL-CX or diRL-CX is used accordingly.

If one of the abovementioned indices X and/or Y is provided with ":Z", this signifies that the respective residue R^{1RL} and/or R^{2RL} is equal to an unbranched, unsubstituted hydrocarbon residue having X-3 or Y-3 carbon atoms having Z double bonds.

Methods for preparing the relevant rhamnolipids are disclosed, for example, in EP2786743 and EP2787065.

Rhamnolipids applicable in the context of the instant invention can also be produced by fermentation of Pseudomonas, especially *Pseudomonas aeruginosa,* which are preferably non genetically modified cells, a technology already disclosed in the eighties, as documented e.g. in EP0282942 and DE4127908. Rhamnolipids produced in *Pseudomonas aeruginosa* cells which have been improved for higher rhamnolipid titres by genetical modification can also be used in the context of the instant invention; such cells have for example been disclosed by Lei et al. in Biotechnol Lett. 2020 Jun;42(6):997-1002.

Rhamnolipids produced by *Pseudomonas aeruginosa* are commercially available from Jeneil Biotech Inc., e.g. under the tradename Zonix,from Logos Technologies (technology acquired by Stepan), e.g. under the tradename NatSurFact, from Biotensidon GmbH, e.g. under the tradename Rhapynal, from AGAE technologies, e.g. under the name R90, R95, R95Md, R95Dd, from Locus Bio-Energy Solutions and from Shanghai Yusheng Industry Co. Ltd., e.g. under the tradename Bio-201 Glycolipids.

The present invention provides a composition preferably comprising as biosurfactant rhamnolipids, characterized in that the biosurfactant component B) comprises
51% by weight to 95% by weight, preferably 55% by weight to 80% by weight, particularly preferably 60% by weight to 70% by weight, of diRL-C10C10 and
where the percentages by weight refer to the sum of all of the rhamnolipids present.

A preferred composition according to the invention is characterized in that the composition comprises as biosurfactant rhamnolipids as described above with a content of
0.5% by weight to 15% by weight, preferably 3% by weight to 12% by weight, particularly preferably 5% by weight to 10% by weight, of diRL-C10C12:1,
where the percentages by weight refer to the sum of all of the rhamnolipids present.

A further preferred composition according to the invention is characterized in that the composition comprises as biosurfactant rhamnolipids as described above with a content of
0.5 to 25% by weight, preferably 5% by weight to 15% by weight, particularly preferably 7% by weight to 12% by weight, of diRL-C10C12,
where the percentages by weight refer to the sum of all of the rhamnolipids present.

A further preferred composition according to the invention is characterized in that the composition comprises as biosurfactant rhamnolipids as described above with a content of
0.1% by weight to 25% by weight, preferably 2% by weight to 10% by weight, particularly preferably 4% by weight to 8% by weight, of diRL-C8C10,
where the percentages by weight refer to the sum total of all rhamnolipids present.

An even further preferred composition according to the invention is characterized in that the composition comprises as biosurfactant rhamnolipids as described above with a content of
0.1% by weight to 5% by weight, preferably 0.5% by weight to 3% by weight, particularly preferably 0.5% by weight to 2% by weight, of monoRL-C8C10 and/or, preferably and
0.1% by weight to 5% by weight, preferably 0.5% by weight to 3% by weight, particularly preferably 0.5% by weight to 2% by weight, of monoRL-C10C10,
where the percentages by weight refer to the sum of all of the rhamnolipids present.

In the context of the present invention, the term "sophorolipids" preferably is understood as meaning compounds of the general formulae (IIa) and (IIb) and salts thereof 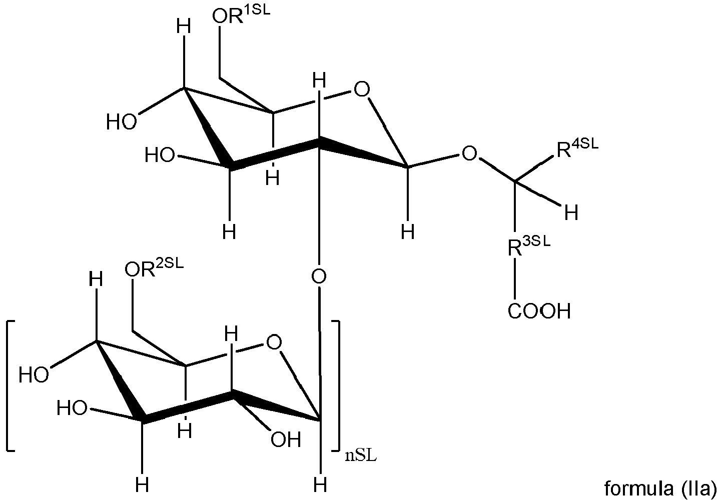 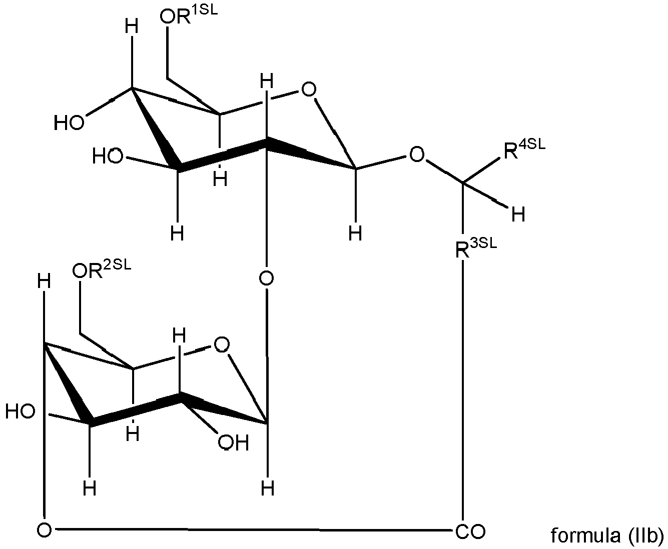 where
- R^{1SL} =: H or CO-CHs,
- R^{2SL} =: H or CO-CHs,
- R^{3SL} =: a divalent organic moiety which comprises 6 to 32 carbon atoms and which is unsubstituted or substituted by hydroxyl functions, is unbranched and optionally comprises one to three double or triple bonds,
- R^{4SL} =: H, CH₃ or a monovalent organic radical which comprises 2 to 10 carbon atoms and which is unsubstituted or substituted by hydroxyl functions, which is unbranched and which optionally comprises one to three double or triple bonds, and
- nSL =: 1 or 0.

Sophorolipids may be used in accordance with the invention in their acid form or their lactone form. Preferred compositions according to the instant invention comprise a sophorolipid in which the ratio by weight of lactone form to acid form is in the range of 20:80 to 80:20, especially preferably in the ranges of 30:70 to 40:60.

To determine the content of sophorolipids in the acid or lactone form in a formulation, refer to EP1411111 B1, page 8, paragraph [0053].

Preferably contained sophorolipids in the composition according to the instant invention are compounds of the general formulae (Ila) and (Ilb)
in which
- R^{1SL} =: H or CO-CHs,
- R^{2SL} =: H or CO-CHs,

R^{3SL} = a divalent organic moiety which comprises 6 to 32, preferably 7 to 19 carbon atoms and which is unsubstituted or substituted by hydroxyl functions, is unbranched and optionally comprises one to three double or triple bonds,
R^{4SL} = H, CH₃ or a monovalent organic radical which comprises 2 to 10 carbon atoms and which is unsubstituted or substituted by hydroxyl functions, which is unbranched and which optionally comprises one to three double or triple bonds, and

- n =: 0 or 1,
in particular of those compounds of the general formulae (Ila) and (IIb)
in which
- R^{1SL} =: H or CO-CHs,
- R^{2SL} =: H or CO-CHs,
R^{3SL} = a divalent organic moiety which comprises 6 to 32, preferably 7 to 19 carbon atoms and which is unsubstituted or substituted by hydroxyl functions, is unbranched and optionally comprises one to three double or triple bonds,
- R^{4SL} =: H, CH₃ or C₉H₁₉, and
- nSL =: 0 or 1,
and very especially preferably compounds of the general formulae (Ila) and (IIb) in which
- R^{1SL} =: H or CO-CHs,
- R^{2SL} =: H or CO-CHs,
R^{3SL} = a divalent organic moiety which comprises 6 to 32, preferably 7 to 19 carbon atoms and which is unsubstituted or substituted by hydroxyl functions, is unbranched and optionally comprises one to three double or triple bonds, in particular C₈H₁₅=C₇H₁₄,
- R^{4SL} =: H, CH₃ or C₉H₁₉, in particular H or CH₃, and
- nSL =: 1.

In connection with the present invention, the term "glucolipids" preferably is understood as meaning compounds of the general formula (III) and salts thereof, 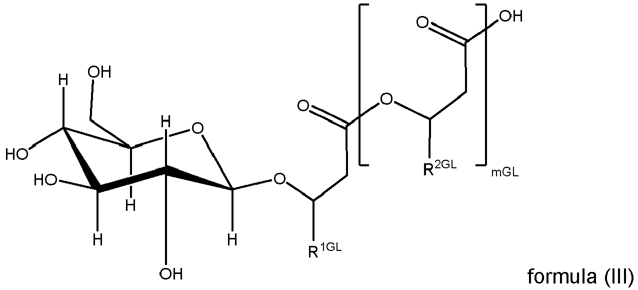 where
mGL = 1 or 0,
R^{1GL} and R^{2GL} = independently of one another identical or different organic radical having 2 to 24 carbon atoms, in particular optionally branched, optionally substituted, in particular hydroxy-substituted, optionally unsaturated, in particular optionally mono-, di- or triunsaturated, alkyl radical, preferably one selected from the group consisting of pentenyl, heptenyl, nonenyl, undecenyl and tridecenyl and (CH₂)ₒ-CH₃ where o = 1 to 23, preferably 4 to 12.

Distinct glucolipids are abbreviated according to the following nomenclature:
"GL-CXCY" is understood as meaning glucolipids of the general formula (III) in which one of the radicals R^{1GL} and R^{2GL} = (CH₂)ₒ-CH₃ where o = X-4 and the remaining radical R^{1GL} or R^{2GL} = (CH₂)ₒ-CH₃ where o = Y-4.

The nomenclature used thus does not differentiate between "CXCY" and "CYCX".

If one of the aforementioned indices X and/or Y is provided with ":Z", then this means that the respective radical R^{1GL} and/or R^{2GL} = an unbranched, unsubstituted hydrocarbon radical with X-3 or Y-3 carbon atoms having Z double bonds.

Methods for production of glucolipids can be carried out as described in WO2019154970.

The compositions according to the instant invention can bear high concentrations of the components to be advantageously useful, e.g. in the production of end customer formulations.

In this context it is preferred in the instant invention, that the composition according to the instant invention is characterized in that it contains component A) in an amount from 0,1 % by weight to 15.0 % by weight, preferably from 1.0 % by weight to 10.0 % by weight, particularly preferably from 2.0 % by weight to 7.0 % by weight, based on the total composition.

It is preferred in the instant invention, that the composition according to the instant invention is characterized in that it contains component A) in an amount from 0.15 4-nitrophenyl octanoate Units / g to 2250 4-nitrophenyl octanoate Units / g, preferably from 1,5 4-nitrophenyl octanoate Units / g to 1500 4-nitrophenyl octanoate Units / g, particularly preferably from 3 4-nitrophenyl octanoate Units / g to 1050 4-nitrophenyl octanoate Units / g, based on the total composition.

The enzymatic activity of component A) in 4-nitrophenyl octanoate Units is determined as described by Gil-Rodriguez et al. in MethodsX; Volume 7, 2020, 100999, whereby 4-nitrophenyl octanoate Unit / g is defined as the converted substrate p-nitrophenol octanoate in µmole per minute and gram of formulation at 25 °C and a pH of 8,5.

It is preferred in the instant invention, that the composition according to the instant invention is characterized in that it contains component B) in an amount from 5.0 % by weight to 80% by weight, preferably from 10.0 % by weight to 75 % by weight, particularly preferably from 25 % by weight to 60 % by weight, based on the total composition.

The composition according to the instant invention preferably comprises at least one non-bio surfactant as component C).

It is preferred in the instant invention, that the composition according to the instant invention is characterized in that it contains component C) in an amount from 1.0 % by weight to 50 % by weight, preferably from 5.0 % by weight to 30 % by weight, particularly preferably from 5.0 % by weight to 20% by weight, based on the total composition.

Preferably the non-bio surfactant contained in the composition according to the instant invention is selected from anionic surfactants, preferably selected from the group comprising, preferably consisting of, anionic, cationic, non-ionic, semi-polar and zwitterionic surfactants, more preferably anionic surfactants.

Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), polyglycerol esters, glycerol esters, propoxylated fatty acid monoethanolamides (PFAM), polyhydroxy alkyl fatty acid amides, or N-acyl N-alkyl derivatives of glucosamine (glucamides, GA, or fatty acid glucamide, FAGA), as well as products available under the trade names SPAN and TWEEN, and combinations thereof.

Non-limiting examples of semipolar surfactants include amine oxides (AO) such as alkyldimethylamineoxide, N-(coco alkyl)-N,N-dimethylamine oxide and N-(tallow-alkyl)-N,N-bis(2-hydroxyethyl)amine oxide, fatty acid alkanolamides and ethoxylated fatty acid alkanolamides, and combinations thereof.

Non-limiting examples of zwitterionic surfactants include betaines, alkyldimethylbetaines, sulfobetaines, and combinations thereof.

The composition according to the instant invention preferably is characterized in that the total sum of all surfactants is from 5.0 % by weight to 90 % by weight, preferably 10.0 % by weight to 80 % by weight, preferably 20 % by weight to 75 % by weight, wherein the percentages by weight relate to the total composition.

It is preferred in the instant invention, that the composition according to the instant invention is characterized in that the sum of all biosurfactant in the total surfactant is from 1 % by weight to 90 % by weight, preferably from 2 % by weight to 80 % by weight, particularly preferably from 51 % by weight to 60% by weight, based on the total amount of surfactant in the composition.

The composition according to the instant invention preferably comprises
D) water.

The composition according to the instant invention preferably has a pH of 4.0 to 12.5, preferably of 5.0 to 10.0, particularly preferably of 5.5 to 9.0.

If the composition according to the instant invention is used, for example, in laundry detergents, it preferably has a pH of 7.0 to 12.5, preferably of 7.5 to 12.0, particularly preferably of 8.0 to 12.0. If the composition according to the instant invention is used, for example, in manual dishwashing agents, it preferably alternatively has a pH of 4 to 8, preferably of 4.5 to 7.5, particularly preferably of 5 to 6.5.

The composition according to the instant invention can further comprise one or more auxiliary agents selected from the group consisting of bleaching systems, hydrotropes, polymers, which may be synthetic, biopolymers, anti-redeposition aids, fiber protection agents, soil release agents, dye transfer inhibitors, fabric hueing agents, opacifiers, blueing dyes, enzyme stabilizing agents, solvents, viscosity modifiers, preservatives, pH-regulators and salts like NaCl and Na2SO4.

Suitable non-aqueous solvents include monohydric or polyhydric alcohols, alkanolamines or glycol ethers, provided they are miscible with water in the specified concentration range.

The solvents are preferably selected from ethanol, n-propanol, i-propanol, butanols, glycol, propanediol, butanediol, glycerine, diglycol, propyldiglycol, butyldiglycol, hexylene glycol, ethylene glycol methyl ether, ethylene glycol ethyl ether, ethylene glycol propyl ether, n-butyl glycol ether, ethylene glycol mono-glycol ether, Diethylene glycol ethyl ether, propylene glycol methyl ether, propylene glycol ethyl ether, Propylene glycol propyl ether, dipropylene glycol monomethyl ether, dipropylene glycol monoethyl ether, diisopropylene glycol monomethyl ether, diisopropylene glycol monoethyl ether, methoxy triglycol, ethoxy triglycol, butoxy triglycol, 1-butoxyethoxy-2-propanol, n-butoxyethoxy-2-propanol, di-butoxyethoxy-2-propanol, 3-butyl-3-methoxyether solvents, and mixtures of these solvents, butoxyethoxy-2-propanol, 3-butyl-3-butyl ether, propyl glycol, di-octanol ether, di-butoxy-2-propanol, 3-butyl-3-butylether, propanol, propylenglycol, di-butoxyethoxy-2-propanol, di-butoxyethoxy-2-propanol, 3-butoxy-3-methoxyether solvents, diisopropylene glycol monomethylether, diisopropylene glycol monomethylether, and mixtures of these solvents. However, it is preferred that the surfactant preparation contains a polyol as the non-aqueous solvent.The polyol can comprise glycerol, 1,2-propanediol, 1,3-propanediol, ethylene glycol, diethylene glycol and / or dipropylene glycol.

Any preservative known in the art for use in detergents may be utilized. Exemplary preservatives include phenoxyethanol, sodium levulinate, sodium benzoate, p-anisic acid, potassium sorbate, benzoic acid, glyceryl caprylate, capryl glycol, penthylene glycol, methyl propane diol, bronopol, isothiazolinone (methylisothiazolinone, chloromethylisothiazolinone) and combinations thereof

Preferably the composition according to the instant invention comprises one or more encapsulate comprising a benefit agent, preferably a sensorial benefit agent. Preferred encapsulates in this context comprise shear/pressure-sensitive action encapsulates, whereby the sensorial benefit agent is released in response to mechanical force (e.g., friction, pressure, shear stress) on the encapsulate. The encapsulate shell is preferably comprised of materials including but not limited to polyurethane, polyamide, polyolefin, polysaccharide, protein, silicone, lipid, modified cellulose, gums, polyacrylate, polyphosphate, polystyrene, polyesters or combinations of these materials. Preferably the sensorial benefit agent comprises a skin benefit agent or an olfactory benefit agent and/or may be a volatile benefit agent. Sensorial benefit agents may also have benefits for hair and/or hard surfaces and/or fabrics. The sensorial benefit may have anti-foam properties, and as such it is advantageous for foaming purposes that it is encapsulated so as not interfering with the foam until release by rubbing. Suitable volatile benefit agents include but are not limited to perfumes, insect repellents, essential oils, sensates such as menthol and aromatherapy actives, preferably perfumes. Mixtures of volatile benefit agents may be used. The total amount of benefit agent is preferably from 0.01 to 10 % by weight, more preferably from 0.05 to 5 % by weight, even more preferably from 0.1 to 4.0 %, most preferably from 0.15 to 4.0 % by weight, based on the total weight of the composition. The preferred benefit agent is a perfume. The composition of the instant invention may also comprise an unconfined (also called non-encapsulated) volatile benefit agent. Where the volatile benefit agent is a perfume, the perfumes described below are suitable for use as the encapsulated volatile benefit agent and also as the unconfined perfume component.

The compositions according to the instant invention can be used in different fields of applications, for example in various cleaning applications.

In this context preferred composition according to the instant inventions are formulations, preferably laundry and dishwashing formulations.

It is preferred in the instant invention, that the formulation according to the instant invention is characterized in that it contains component A) in an amount from 0.001 % by weight to 4.0 % by weight, preferably from 0.01 % by weight to 3.0 % by weight, particularly preferably from 0.1 % by weight to 1.0 % by weight, based on the total composition.

It is preferred in the instant invention, that the formulation according to the instant invention is characterized in that it contains component A) in an amount from 0.0015 4-nitrophenyl octanoate Units / g to 60 4-nitrophenyl octanoate Units / g, preferably from 0.015 4-nitrophenyl octanoate Units / g to 45 4-nitrophenyl octanoate Units / g, particularly preferably from 0.15 4-nitrophenyl octanoate Units / g to 15 4-nitrophenyl octanoate Units / g, based on the total composition.

It is preferred in the instant invention, that the formulation according to the instant invention is characterized in that it contains component B) in an amount from 0.1 % by weight to 25 % by weight, preferably from 3.0 % by weight to 20 % by weight, particularly preferably from 4.0 % by weight to 15 % by weight, based on the total composition.

The composition according to the instant invention is outstanding in its ability to remove oily dirt from a surface.

Therefore, a further aspect of the instant invention is the use of a composition according to the instant invention to remove fat and/or oil-containing stains from a surface, preferably selected from a dish surface or a textile's surface.

In the case of the use according to the invention, particularly preferred embodiments of the compositions according to the instant invention and preferred components thereof are used in the preferred amounts.

The examples adduced hereinafter describe the present invention by way of example, without any intention that the invention, the scope of application of which is apparent from the entirety of the description and the claims, be restricted to the embodiments specified in the examples.

### Examples:

### Example 1: Production of lipase from Stachybotrys chlorohalonata in Pichia pastoris.

The Pichia Expression Kit, original kit, catalogue number K171001 from Thermo Fisher Scientific is used for recombinant expression of Seq.-ID-No. 1.

All steps are carried out as described in the User Guide of the Pichia Expression Kit, Revision A.0 of 2014.

A DNA sequence, codon optimized for expression of Seq.-ID-No. 1 in *Pichia pastoris* was generated using Codon Optimization Tool (ExpOptimizer) (www.novoprolabs.com/tools/codon-optimization), compare Seq.-ID-No. 2.

Seq.-ID-No. 2 is subcloned in to pPIC3.5 via BamH I and Not I including a Kozak-sequence as described in the User Guide and subsequently *Pichia* KM71 is transformed with the Sac I-linearized expression vector to obtain and select His⁺ Mut^{S} transformants as described in the manufacturer's User Guide.

Successful expression of the recombinant lipase according to the User Guide is verified by cell lysates in a propyllaurate assay with thymolphthalein as indicator compared to mock (empty pPIC3.5) transfected KM71 transformants.

### Example 2:

The following formulations are prepared with the numbers given being weight percentages of active matter; lipase is given in 4-nitrophenyl octanoate units per gram total formulation.

Rhamnolipids were prepared as described in EP3023431.

The sophorolipid used is a sophorolipid REWOFERM SL ONE from Evonik, which has a lactone to acid ratio of 40:60.

Glucolipids were produced according to example 2 of WO2019154970 via fermentation.

| Ingredient / Formulation | 1A | 1B | 1C | 1D | 1E | 1F |
|---|---|---|---|---|---|---|
| Rhamnolipids | 14.00 | 14.00 | 14.00 | 14.00 | 14.00 | 14.00 |
| NaOH | 1.90 | 1.90 | 1.90 | 1.90 | 1.90 | 1.90 |
| C12/14 7EO (Dehydol LT7) | 8.50 | 8.50 | 8.50 | 8.50 | 8.50 | 8.50 |
| SLES 2EO (Texapon N70) | - | - | - | - | - | - |
| Sodium xylene sulfonate (Eltesol SX40) | 1.10 | 1.10 | 1.10 | 1.10 | 1.10 | 1.10 |
| Na soap from palm kernel oil | 5.00 | 5.00 | 5.00 | 5.00 | 5.00 | 5.00 |
| Sodium citrate | 3.50 | 3.50 | 3.50 | 3.50 | 3.50 | 3.50 |
| Sodium Phosphonate (Dequest 2066) | 2.37 | 2.37 | 2.37 | 2.37 | 2.37 | 2.37 |
| Ethanol | 2.50 | 2.50 | 2.50 | 2.50 | 2.50 | 2.50 |
| Phenoxyethanol (Acticide SR150) | 0.45 | 0.45 | 0.45 | 0.45 | 0.45 | 0.45 |
| Liquanase.2.5 L | 0.7 | 0.7 | 0.7 | 0.7 | 0.7 | 0.7 |
| Amplify Prime 100 L | 0.4 | 0.4 | 0.4 | 0.4 | 0.4 | 0.4 |
| Lipase from example 1 | 6 U/g | | | | | |
| Lipex 100 L Evity (Novozymes) | | 6 U/g | | | | |
| Biolipasa L (Biokatal) | | | 6 U/g | | | |
| Lipase OF (Meito Sangyo) | | | | 6 U/g | | |
| Addclean LP L (advanced enzymes) | | | | | 6 U/g | |
| Meteolipase (Meteoric Life Sciences) | | | | | | 6 U/g |
| Mannaway 4.0 L | 0.2 | 0.2 | 0.2 | 0.2 | 0.2 | 0.2 |
| Water | ad 100 | ad 100 | ad 100 | ad 100 | ad 100 | ad 100 |
| pH | 8.4 | 8.4 | 8.4 | 8.4 | 8.4 | 8.4 |

| Ingredient / Formulation | 2A | 2B | 2C | 2D | 2E | 2F |
|---|---|---|---|---|---|---|
| Sophorolipids | 14.00 | 14.00 | 14.00 | 14.00 | 14.00 | 14.00 |
| NaOH | 1.90 | 1.90 | 1.90 | 1.90 | 1.90 | 1.90 |
| C12/14 7EO (Dehydol LT7) | 8.50 | 8.50 | 8.50 | 8.50 | 8.50 | 8.50 |
| SLES 2EO (Texapon N70) | - | - | - | - | - | - |
| Sodium xylene sulfonate (Eltesol SX40) | 1.10 | 1.10 | 1.10 | 1.10 | 1.10 | 1.10 |
| Na soap from palm kernel oil | 5.00 | 5.00 | 5.00 | 5.00 | 5.00 | 5.00 |
| Sodium citrate | 3.50 | 3.50 | 3.50 | 3.50 | 3.50 | 3.50 |
| Sodium Phosphonate (Dequest 2066) | 2.37 | 2.37 | 2.37 | 2.37 | 2.37 | 2.37 |
| Ethanol | 2.50 | 2.50 | 2.50 | 2.50 | 2.50 | 2.50 |
| Phenoxyethanol (Acticide SR150) | 0.45 | 0.45 | 0.45 | 0.45 | 0.45 | 0.45 |
| Liquanase.2.5 L | 0.7 | 0.7 | 0.7 | 0.7 | 0.7 | 0.7 |
| Amplify Prime 100 L | 0.4 | 0.4 | 0.4 | 0.4 | 0.4 | 0.4 |
| Lipase from example 1 | 6 U/g | | | | | |
| Lipex 100 L Evity (Novozymes) | | 6 U/g | | | | |
| Biolipasa L (Biokatal) | | | 6 U/g | | | |
| Lipase OF (Meito Sangyo) | | | | 6 U/g | | |
| Addclean LP L (advanced enzymes) | | | | | 6 U/g | |
| Meteolipase (Meteoric Life Sciences) | | | | | | 6 U/g |
| Mannaway 4.0 L | 0.2 | 0.2 | 0.2 | 0.2 | 0.2 | 0.2 |
| Water | ad 100 | ad 100 | ad 100 | ad 100 | ad 100 | ad 100 |
| pH | 8.4 | 8.4 | 8.4 | 8.4 | 8.4 | 8.4 |

| Ingredient / Formulation | 3A | 3B | 3C | 3D | 3E | 3F |
|---|---|---|---|---|---|---|
| Glucolipids | 14.00 | 14.00 | 14.00 | 14.00 | 14.00 | 14.00 |
| NaOH | 1.90 | 1.90 | 1.90 | 1.90 | 1.90 | 1.90 |
| C12/14 7EO (Dehydol LT7) | 8.50 | 8.50 | 8.50 | 8.50 | 8.50 | 8.50 |
| SLES 2EO (Texapon N70) | - | - | - | - | - | - |
| Sodium xylene sulfonate (Eltesol SX40) | 1.10 | 1.10 | 1.10 | 1.10 | 1.10 | 1.10 |
| Na soap from palm kernel oil | 5.00 | 5.00 | 5.00 | 5.00 | 5.00 | 5.00 |
| Sodium citrate | 3.50 | 3.50 | 3.50 | 3.50 | 3.50 | 3.50 |
| Sodium Phosphonate (Dequest 2066) | 2.37 | 2.37 | 2.37 | 2.37 | 2.37 | 2.37 |
| Ethanol | 2.50 | 2.50 | 2.50 | 2.50 | 2.50 | 2.50 |
| Phenoxyethanol (Acticide SR150) | 0.45 | 0.45 | 0.45 | 0.45 | 0.45 | 0.45 |
| Liquanase.2.5 L | 0.7 | 0.7 | 0.7 | 0.7 | 0.7 | 0.7 |
| Amplify Prime 100 L | 0.4 | 0.4 | 0.4 | 0.4 | 0.4 | 0.4 |
| Lipase from example 1 | 6 U/g | | | | | |
| Preferenz L 100 (essential ingredients) | | 6 U/g | | | | |
| Biolipasa L (Biokatal) | | | 6 U/g | | | |
| Lipase OF (Meito Sangyo) | | | | 6 U/g | | |
| Addclean LP L (advanced enzymes) | | | | | 6 U/g | |
| Meteolipase (Meteoric Life Sciences) | | | | | | 6 U/g |
| Mannaway 4.0 L | 0.2 | 0.2 | 0.2 | 0.2 | 0.2 | 0.2 |
| Water | ad 100 | ad 100 | ad 100 | ad 100 | ad 100 | ad 100 |
| pH | 8.4 | 8.4 | 8.4 | 8.4 | 8.4 | 8.4 |

For the evaluation of the formulations above a stain removal test is carried out 2 or 3 cycles for the stain removal and is done for 8 fat stains, following the A.I.S.E test (reduced). The test s performed at 40°C.

In this performance test two cycles (replicates) are considered for the compositions.

The water used had a water hardness of 14 ° deutsche Haerte.

A programmable Miele electronic household washing machine is used. Fuzzy logic type control is disabled. The washing program is listed are explained in the following table:

| | Cotton wash program (at 40°C) |
|---|---|
| Duration Main Wash | 70 min |
| Total Program Duration | 120 min |
| Water Quantity Main Wash | (15 ± 2) L |
| Total Water Quantity | (55 ± 5) L |
| Number of Rinse Cycles | 3 |
| Final Spin Speed | 1200 rpm |

3kg of standardized cotton load is previously washed at 60°C with ECE2 detergent without bleach.

A set of fatty stains set for each replicate is used. The different stains are shown below.

| Stains | |
|---|---|
| Lipstick diluted red (instead of another make up stain) | Grease. oil/Greasy - Enzymatic |
| Carbon black/ olive oil | Grease. oil/Greasy |
| IEC carbon black/mineral oil | Grease. oil/Greasy |
| Butterfat with colorant | Grease. oil/Greasy - Enzymatic |
| High discriminative sebum | Grease. oil/Greasy - Enzymatic |
| Olive oil with chlorophyl | Grease. oil/Greasy - Enzymatic |
| Beef fat. colored with sudan red | Grease. oil/Greasy - Enzymatic |
| 50%lard. 50%Beef fat with colorant | Grease. oil/Greasy - Enzymatic |

Together with the 3kg of ballast load and two new units of soil SBL2004. the 8 fat stains are introduced in each wash cycle. SBL2004 is the 'standard' Soil Ballast from wfk; 100 % cotton. Approximative 8 g soil/swatch are used for testing laundry processes. 50 g of each composition is used in the washing process.

The evaluation of the degree of stain removal is carried out measuring the reflectance via spectrophotometer. using the Y-value of the Y. x. y color coordinates measurement. light source D65 with a UV cut-off filter at 420 nm. Aperture used for real stains 15 mm (minimum 12 mm). Stains are measured unfolded. 2 measurements per stain (in the center of the circular area. or closest homogenous area). Measurements are taken on each stain before wash (to verify quality of stains) and after the wash and to evaluate standard deviations and are reported.

Results are expressed as the mean of value Y between two replicates and shown below. The better, the larger the Y value.

| Stain | 1A | 1B | 1C | 1D | 1E | 1F |
|---|---|---|---|---|---|---|
| | Average | Average | Average | Average | Average | Average |
| Lipstick diluted red | 68.8 | 62.6 | 60.6 | 61.3 | 61.8 | 63.4 |
| Carbon black/olive oil | 39.7 | 33.4 | 31.4 | 32.6 | 33.1 | 30.9 |
| IEC carbon black/mineral oil | 43.3 | 35.9 | 34.7 | 36.1 | 34.8 | 33.5 |
| Butterfat with colourant | 74.6 | 68.0 | 69.8 | 67.6 | 68.4 | 65.4 |
| High discriminative sebum | 66.4 | 64.2 | 62.6 | 61.2 | 59.9 | 60.5 |
| Olive oil with chlorophyl | 57.5 | 54.8 | 55.1 | 55.2 | 54.9 | 55.0 |
| Beef fat. coloured with sudan red | 46.9 | 41.7 | 40.1 | 39.8 | 43.2 | 36.9 |
| 50%lard. 50%Beef fat with colorant | 57.1 | 54.1 | 50.3 | 52.6 | 53.6 | 50.2 |

| Stain | 2A | 2B | 2C | 2D | 2E | 2F |
|---|---|---|---|---|---|---|
| | Average | Average | Average | Average | Average | Average |
| Lipstick diluted red | 67.1 | 60.2 | 58.8 | 60.1 | 60.3 | 60.5 |
| Carbon black/olive oil | 37.4 | 30.3 | 29.6 | 30.2 | 30.9 | 28.1 |
| IEC carbon black/mineral oil | 41.3 | 33.5 | 32.2 | 34.8 | 32.8 | 32.0 |
| Butterfat with colourant | 73.7 | 65.7 | 67.7 | 65.5 | 67.0 | 63.6 |
| High discriminative sebum | 65.1 | 63.8 | 60.4 | 59.8 | 58.5 | 58.7 |
| Olive oil with chlorophyl | 55.7 | 51.9 | 52.9 | 54.2 | 52.6 | 53.04 |
| Beef fat. coloured with sudan red | 44.9 | 39.4 | 37.7 | 36.9 | 40.8 | 36.0 |
| 50%lard. 50%Beef fat with colorant | 55.3 | 50.9 | 48.5 | 50.4 | 52.2 | 49.6 |

| Stain | 3A | 3B | 3C | 3D | 3E | 3F |
|---|---|---|---|---|---|---|
| | Average | Average | Average | Average | Average | Average |
| Lipstick diluted red | 69.1 | 63.2 | 60.6 | 61.9 | 61.8 | 63.4 |
| Carbon black/olive oil | 40.7 | 35.6 | 31.4 | 34.1 | 33.1 | 30.9 |
| IEC carbon black/mineral oil | 45.6 | 37.4 | 34.7 | 38.2 | 34.8 | 33.5 |
| Butterfat with colourant | 76.1 | 70.5 | 69.8 | 68.3 | 68.4 | 65.4 |
| High discriminative sebum | 67.3 | 66.8 | 62.6 | 63.4 | 59.9 | 60.5 |
| Olive oil with chlorophyl | 58.4 | 56.3 | 55.1 | 56.6 | 54.9 | 55.0 |
| Beef fat. coloured with sudan red | 46.7 | 43.4 | 40.1 | 41.0 | 43.2 | 36.9 |
| 50%lard. 50%Beef fat with colorant | 58.3 | 56.9 | 50.3 | 53.6 | 53.6 | 50.2 |

As can be seen ion the results, the lipase of example 1 outperforms all commercially available lipases on all fatty stains, no matter what biosurfactant is used as the main surfactant in the detergent.

### Further formulation examples of laundry pre-spotter

| | Example 1 | Example 2 | Example 3 | Example 4 |
|---|---|---|---|---|
| Ingredient | [%] | [%] | [%] | [%] |
| Rhamnolipids | 5 | 12 | 5 | 5 |
| Sophorolipids | 12 | 5 | 1 | 1 |
| Lauryl/Myristil Glucoside | | | 12 | 12 |
| Propylene Glycol | 2 | 2 | 2 | 2 |
| Na citrate | 2 | 2 | 2 | 2 |
| Na formate | 0.5 | 0.5 | 0.5 | 0.5 |
| CaCl2 | 0.1 | 0.1 | 0.1 | 0.1 |
| NaOH | 0.05 | 0.05 | 0.05 | 0.05 |
| Protease | 1 | 1 | | 0.4 |
| Amylase | 0.4 | 0.4 | | 0.2 |
| Lipase from example 1 | 6 U/g | 6 U/g | 6 U/g | 6 U/g |
| Mannanase | 0.2 | 0.2 | | 0.2 |
| Pectinase | 0.2 | 0.2 | | 0.2 |
| Cellulase | 0.2 | 0.2 | | 0.2 |
| Water | ad 100 | ad 100 | ad 100 | ad 100 |

| | Example 5 | Example 6 | Example 7 | Example 8 |
|---|---|---|---|---|
| Ingredient | [%] | [%] | [%] | [%] |
| Rhamnolipids | 5 | 3 | 5 | 1 |
| Lauryl/Myristil Glucoside | 12 | 9 | | |
| Propylene Glycol | 2 | 2 | 2 | 2 |
| Na citrate | 2 | 2 | 2 | 2 |
| Na formate | 0.5 | 0.5 | 0.5 | 0.5 |
| CaCl2 | 0.1 | 0.1 | 0.1 | 0.1 |
| NaOH | 0.05 | 0.05 | 0.05 | 0.05 |
| Protease | 1 | 1 | 1 | 1 |
| Amylase | 0.4 | 0.4 | 0.4 | 0.4 |
| Lipase from example 1 | 6 U/g | 6 U/g | 6 U/g | 6 U/g |
| Mannanase | 0.2 | 0.2 | 0.2 | 0.2 |
| Pectinase | 0.2 | 0.2 | 0.2 | 0.2 |
| Cellulase | 0.2 | 0.2 | 0.2 | 0.2 |
| Water | ad 100 | ad 100 | ad 100 | ad 100 |

*Further formulation examples of laundry detergents*

| Ingredient / Formulation | 4A | 4B | 4C | 4D | 4E | 4F |
|---|---|---|---|---|---|---|
| Rhamnolipids | 14.00 | 7.0 | 7.0 | 7.0 | 7.0 | 7.0 |
| Glucolipids | - | - | - | - | - | - |
| Sophorolipids | - | - | - | - | - | - |
| C12/14 7EO (Dehydol^{®} LT7) | - | 5.0 | 5.0 | 2.5 | - | 5.0 |
| SLES 2EO (Texapon^{®} N70) | - | 7.0 | 5.0 | 2.5 | 5.0 | .0 |
| Linear alkylbenzene Sulfonates (LAS) | - | - | .5 | 5 | - | - |
| C8C16 Alkyl polyglucosid (Glucopon^{®} 650 EC) | - | - | - | - | 2.5 | - |
| Amine oxide | - | - | - | 1 | - | - |
| Sodium xylene sulfonate (Eltesol^{®} SX40) | 1.0 | 1.0 | 1.0 | 1.0 | 1.0 | 1.0 |
| Na soap from palm kernel oil | 5.00 | 5.00 | 5.00 | 5.00 | 5.00 | 5.00 |
| Amide polyglycol ether (Amidet^{®} N) | - | - | - | - | - | 1.0 |
| Polyethylene imine PEl, 20 EO (Sokalan^{®} HP20) | 1.0 | 1.0 | 1.0 | 1.0 | 1.0 | 1.0 |
| Vinylpyrrolidone/Vinylimidazole copolymer (Sokalan^{®} HP 56) | - | - | - | - | - | - |
| Texcare^{®} SRN 170 | - | - | - | - | - | - |
| Sodium citrate | 3.50 | 3.50 | 3.50 | 3.50 | 3.50 | 3.50 |
| Sodium Phosphonate (Dequest^{®} 2066) | 2.0 | 2.0 | 2.0 | 2.0 | 2.0 | 2.0 |
| Ethanol | 2.50 | 2.50 | 2.50 | 2.50 | 2.50 | 2.50 |
| MPG | 1.0 | 1.0 | 1.0 | 1.0 | 1.0 | 1.0 |
| Boric acid | 1.0 | 1.0 | 1.0 | 1.0 | 1.0 | 1.0 |
| Phenoxyethanol (Acticide^{®} SR150) | 0.45 | 0.45 | 0.45 | 0.45 | 0.45 | 0.45 |
| Liquanase^{®} 3.5 L | 1.0 | 1.0 | 1.0 | 1.0 | 1.0 | 1.0 |
| Amplify^{®} Prime 100 L | 0.4 | 0.4 | 0.4 | 0.4 | 0.4 | 0.4 |
| Lipase from example 1 | 6 U/g | 6 U/g | 6 U/g | 6 U/g | 6 U/g | 6 U/g |
| Mannaway^{®} 200 L | 0.2 | 0.2 | 0.2 | 0.2 | 0.2 | 0.2 |
| Carezyme^{®} Premium 4500 L | 0.2 | 0.2 | 0.2 | 0.2 | 0.2 | 0.2 |
| XPect^{®} 1000 L | 0.2 | 0.2 | 0.2 | 0.2 | 0.2 | 0.2 |
| Celluclean^{®} 5000 L | 0.2 | 0.2 | 0.2 | 0.2 | 0.2 | 0.2 |
| Pristine^{®} 100 L | 0.1 | 0.1 | 0.1 | 0.1 | 0.1 | 0.1 |
| NaOH | 1.0 | 1.0 | 1.0 | 1.0 | 1.0 | 1.0 |
| Water | ad 100 | ad 100 | ad 100 | ad 100 | ad 100 | ad 100 |
| pH | 8.4 | 8.4 | 8.4 | 8.4 | 8.4 | 8.4 |

| Ingredient / Formulation | 5A | 5B | 5C | 5D | 5E | 5F |
|---|---|---|---|---|---|---|
| Rhamnolipids | 14.00 | 7.0 | 7.0 | 7.0 | 7.0 | 7.0 |
| Glucolipids | - | - | - | - | - | - |
| Sophorolipids | - | - | - | - | - | - |
| C12/14 7EO (Dehydol^{®} LT7) | - | 5.0 | 5.0 | 2.5 | | 5.0 |
| SLES 2EO (Texapon^{®} N70) | - | 7.0 | 5.0 | 2.5 | 5.0 | .0 |
| Linear alkylbenzene Sulfonates (LAS) | - | - | .5 | 5 | - | - |
| C8C16 Alkyl polyglucosid (Glucopon^{®} 650 EC) | - | - | - | - | 2.5 | - |
| Amine oxide | - | - | - | 1 | - | - |
| Sodium xylene sulfonate (Eltesol^{®} SX40) | 1.0 | 1.0 | 1.0 | 1.0 | 1.0 | 1.0 |
| Na soap from palm kernel oil | 5.00 | 5.00 | 5.00 | 5.00 | 5.00 | 5.00 |
| Amide polyglycol ether (Amidet^{®} N) | - | - | - | - | - | 1.0 |
| Polyethylene imine PEl, 20 EO (Sokalan^{®} HP20) | - | - | - | - | - | - |
| Vinylpyrrolidone/Vinylimidazole copolymer (Sokalan^{®} HP 56) | 2.0 | 2.0 | 2.0 | 2.0 | 2.0 | 2.0 |
| Texcare^{®} SRN 170 | - | - | - | - | - | - |
| Sodium citrate | 3.50 | 3.50 | 3.50 | 3.50 | 3.50 | 3.50 |
| Sodium Phosphonate (Dequest^{®} 2066) | 2.0 | 2.0 | 2.0 | 2.0 | 2.0 | 2.0 |
| Ethanol | 2.50 | 2.50 | 2.50 | 2.50 | 2.50 | 2.50 |
| MPG | 1.0 | 1.0 | 1.0 | 1.0 | 1.0 | 1.0 |
| Boric acid | 1.0 | 1.0 | 1.0 | 1.0 | 1.0 | 1.0 |
| Phenoxyethanol (Acticide^{®} SR150) | 0.45 | 0.45 | 0.45 | 0.45 | 0.45 | 0.45 |
| Liquanase^{®} 3.5 L | 1.0 | 1.0 | 1.0 | 1.0 | 1.0 | 1.0 |
| Amplify^{®} Prime 100 L | 0.4 | 0.4 | 0.4 | 0.4 | 0.4 | 0.4 |
| Lipase from example 1 | 6 U/g | 6 U/g | 6 U/g | 6 U/g | 6 U/g | 6 U/g |
| Mannaway^{®} 200 L | 0.2 | 0.2 | 0.2 | 0.2 | 0.2 | 0.2 |
| Carezyme^{®} Premium 4500 L | 0.2 | 0.2 | 0.2 | 0.2 | 0.2 | 0.2 |
| XPect^{®} 1000 L | 0.2 | 0.2 | 0.2 | 0.2 | 0.2 | 0.2 |
| Celluclean^{®} 5000 L | 0.2 | 0.2 | 0.2 | 0.2 | 0.2 | 0.2 |
| Pristine^{®} 100 L | 0.1 | 0.1 | 0.1 | 0.1 | 0.1 | 0.1 |
| NaOH | 1.0 | 1.0 | 1.0 | 1.0 | 1.0 | 1.0 |
| Water | add 100 | ad 100 | ad 100 | ad 100 | ad 100 | ad 100 |
| pH | 8.4 | 8.4 | 8.4 | 8.4 | 8.4 | 8.4 |

| Ingredient / Formulation | 6A | 6B | 6C | 6D | 6E | 6F |
|---|---|---|---|---|---|---|
| Rhamnolipids | 14.00 | 7.0 | 7.0 | 7.0 | 7.0 | 7.0 |
| Glucolipids | - | - | - | - | - | - |
| Sophorolipids | - | - | - | - | - | - |
| C12/14 7EO (Dehydol^{®} LT7) | - | 5.0 | 5.0 | 2.5 | - | 5.0 |
| SLES 2EO (Texapon^{®} N70) | - | 7.0 | 5.0 | 2.5 | 5.0 | .0 |
| Linear alkylbenzene Sulfonates (LAS) | - | - | .5 | 5 | - | - |
| C8C16 Alkyl polyglucosid (Glucopon^{®} 650 EC) | - | - | - | - | 2.5 | - |
| Amine oxide | - | - | - | 1 | - | - |
| Sodium xylene sulfonate (Eltesol^{®} SX40) | 1.0 | 1.0 | 1.0 | 1.0 | 1.0 | 1.0 |
| Na soap from palm kernel oil | 5.00 | 5.00 | 5.00 | 5.00 | 5.00 | 5.00 |
| Amide polyglycol ether (Amidet^{®} N) | - | - | - | - | - | 1.0 |
| Polyethylene imine PEl, 20 EO (Sokalan^{®} HP20) | - | - | - | - | - | - |
| Vinylpyrrolidone/Vinylimidazole copolymer (Sokalan^{®} HP 56) | - | - | - | - | - | - |
| Texcare^{®} SRN 170 | 2.0 | 2.0 | 2.0 | 2.0 | 2.0 | 2.0 |
| Sodium citrate | 3.50 | 3.50 | 3.50 | 3.50 | 3.50 | 3.50 |
| Sodium Phosphonate (Dequest^{®} 2066) | 2.0 | 2.0 | 2.0 | 2.0 | 2.0 | 2.0 |
| Ethanol | 2.50 | 2.50 | 2.50 | 2.50 | 2.50 | 2.50 |
| MPG | 1.0 | 1.0 | 1.0 | 1.0 | 1.0 | 1.0 |
| Boric acid | 1.0 | 1.0 | 1.0 | 1.0 | 1.0 | 1.0 |
| Phenoxyethanol (Acticide^{®} SR150) | 0.45 | 0.45 | 0.45 | 0.45 | 0.45 | 0.45 |
| Liquanase^{®} 3.5 L | 1.0 | 1.0 | 1.0 | 1.0 | 1.0 | 1.0 |
| Amplify^{®} Prime 100 L | 0.4 | 0.4 | 0.4 | 0.4 | 0.4 | 0.4 |
| Lipase from example 1 | 6 U/g | 6 U/g | 6 U/g | 6 U/g | 6 U/g | 6 U/g |
| Mannaway^{®} 200 L | 0.2 | 0.2 | 0.2 | 0.2 | 0.2 | 0.2 |
| Carezyme^{®} Premium 4500 L | 0.2 | 0.2 | 0.2 | 0.2 | 0.2 | 0.2 |
| XPect^{®} 1000 L | 0.2 | 0.2 | 0.2 | 0.2 | 0.2 | 0.2 |
| Celluclean^{®} 5000 L | 0.2 | 0.2 | 0.2 | 0.2 | 0.2 | 0.2 |
| Pristine^{®} 100 L | 0.1 | 0.1 | 0.1 | 0.1 | 0.1 | 0.1 |
| NaOH | 1.0 | 1.0 | 1.0 | 1.0 | 1.0 | 1.0 |
| Water | ad 100 | ad 100 | ad 100 | ad 100 | ad 100 | ad 100 |
| pH | 8.4 | 8.4 | 8.4 | 8.4 | 8.4 | 8.4 |

| Ingredient / Formulation | 7A | 7B | 7C | 7D | 7E | 7F |
|---|---|---|---|---|---|---|
| Rhamnolipids | 14.00 | 7.0 | 7.0 | 7.0 | 7.0 | 7.0 |
| Glucolipids | - | - | - | - | - | - |
| Sophorolipids | 1.0 | 1.0 | 1.0 | 1.0 | 1.0 | 1.0 |
| C12/14 7EO (Dehydol^{®} LT7) | - | 5.0 | 5.0 | 2.5 | - | 5.0 |
| SLES 2EO (Texapon^{®} N70) | - | 7.0 | 5.0 | 2.5 | 5.0 | .0 |
| Linear alkylbenzene Sulfonates (LAS) | - | - | .5 | 5 | - | - |
| C8C16 Alkyl polyglucosid (Glucopon^{®} 650 EC) | - | - | - | - | 2.5 | - |
| Amine oxide | - | - | - | 1 | - | - |
| Sodium xylene sulfonate (Eltesol^{®} SX40) | 1.0 | 1.0 | 1.0 | 1.0 | 1.0 | 1.0 |
| Na soap from palm kernel oil | 5.00 | 5.00 | 5.00 | 5.00 | 5.00 | 5.00 |
| Amide polyglycol ether (Amidet^{®} N) | - | - | - | - | - | 1.0 |
| Polyethylene imine PEl, 20 EO (Sokalan^{®} HP20) | - | - | - | - | - | - |
| Vinylpyrrolidone/Vinylimidazole copolymer (Sokalan^{®} HP 56) | - | - | - | - | - | - |
| Texcare^{®} SRN 170 | 2.0 | 2.0 | 2.0 | 2.0 | 2.0 | 2.0 |
| Sodium citrate | 3.50 | 3.50 | 3.50 | 3.50 | 3.50 | 3.50 |
| Sodium Phosphonate (Dequest^{®} 2066) | 2.0 | 2.0 | 2.0 | 2.0 | 2.0 | 2.0 |
| Ethanol | 2.50 | 2.50 | 2.50 | 2.50 | 2.50 | 2.50 |
| MPG | 1.0 | 1.0 | 1.0 | 1.0 | 1.0 | 1.0 |
| Phenoxyethanol (Acticide^{®} SR150) | 0.45 | 0.45 | 0.45 | 0.45 | 0.45 | 0.45 |
| Liquanase^{®} 3.5 L | 1.0 | 1.0 | 1.0 | 1.0 | 1.0 | 1.0 |
| Amplify^{®} Prime 100 L | 0.4 | 0.4 | 0.4 | 0.4 | 0.4 | 0.4 |
| Lipase from example 1 | 6 U/g | 6 U/g | 6 U/g | 6 U/g | 6 U/g | 6 U/g |
| Mannaway^{®} 200 L | 0.2 | 0.2 | 0.2 | 0.2 | 0.2 | 0.2 |
| Carezyme^{®} Premium 4500 L | 0.2 | 0.2 | 0.2 | 0.2 | 0.2 | 0.2 |
| XPect^{®} 1000 L | 0.2 | 0.2 | 0.2 | 0.2 | 0.2 | 0.2 |
| Celluclean^{®} 5000 L | 0.2 | 0.2 | 0.2 | 0.2 | 0.2 | 0.2 |
| Pristine^{®} 100 L | 0.1 | 0.1 | 0.1 | 0.1 | 0.1 | 0.1 |
| NaOH | 1.0 | 1.0 | 1.0 | 1.0 | 1.0 | 1.0 |
| Water | ad 100 | ad 100 | ad 100 | ad 100 | ad 100 | ad 100 |
| pH | 8.4 | 8.4 | 8.4 | 8.4 | 8.4 | 8.4 |

| Ingredient / Formulation | 8A | 8B | 8C | 8D | 8E | 8F |
|---|---|---|---|---|---|---|
| Rhamnolipids | - | - | - | - | - | - |
| Glucolipids | 14.00 | 7.0 | 7.0 | 7.0 | 7.0 | 7.0 |
| Sophorolipids | - | - | - | - | - | - |
| C12/14 7EO (Dehydol^{®} LT7) | - | 5.0 | 5.0 | 2.5 | - | 5.0 |
| SLES 2EO (Texapon^{®} N70) | - | 7.0 | 5.0 | 2.5 | 5.0 | .0 |
| Linear alkylbenzene Sulfonates (LAS) | - | - | .5 | 5 | - | - |
| C8C16 Alkyl polyglucosid (Glucopon^{®} 650 EC) | - | - | - | - | 2.5 | - |
| Amine oxide | - | - | - | 1 | - | - |
| Sodium xylene sulfonate (Eltesol^{®} SX40) | 1.0 | 1.0 | 1.0 | 1.0 | 1.0 | 1.0 |
| Na soap from palm kernel oil | 5.00 | 5.00 | 5.00 | 5.00 | 5.00 | 5.00 |
| Amide polyglycol ether (Amidet^{®} N) | - | - | - | - | - | 1.0 |
| Polyethylene imine PEl, 20 EO (Sokalan^{®} HP20) | - | - | - | - | - | - |
| Vinylpyrrolidone/Vinylimidazole copolymer (Sokalan^{®} HP 56) | - | - | - | - | - | - |
| Texcare^{®} SRN 170 | 2.0 | 2.0 | 2.0 | 2.0 | 2.0 | 2.0 |
| Sodium citrate | 3.50 | 3.50 | 3.50 | 3.50 | 3.50 | 3.50 |
| Sodium Phosphonate (Dequest^{®} 2066) | 2.0 | 2.0 | 2.0 | 2.0 | 2.0 | 2.0 |
| Ethanol | 2.50 | 2.50 | 2.50 | 2.50 | 2.50 | 2.50 |
| MPG | 1.0 | 1.0 | 1.0 | 1.0 | 1.0 | 1.0 |
| Phenoxyethanol (Acticide^{®} SR150) | 0.45 | 0.45 | 0.45 | 0.45 | 0.45 | 0.45 |
| Liquanase^{®} 3.5 L | 1.0 | 1.0 | 1.0 | 1.0 | 1.0 | 1.0 |
| Amplify^{®} Prime 100 L | 0.4 | 0.4 | 0.4 | 0.4 | 0.4 | 0.4 |
| Lipase from example 1 | 6 U/g | 6 U/g | 6 U/g | 6 U/g | 6 U/g | 6 U/g |
| Mannaway^{®} 200 L | 0.2 | 0.2 | 0.2 | 0.2 | 0.2 | 0.2 |
| Carezyme^{®} Premium 4500 L | 0.2 | 0.2 | 0.2 | 0.2 | 0.2 | 0.2 |
| XPect^{®} 1000 L | 0.2 | 0.2 | 0.2 | 0.2 | 0.2 | 0.2 |
| Celluclean^{®} 5000 L | 0.2 | 0.2 | 0.2 | 0.2 | 0.2 | 0.2 |
| Pristine^{®} 100 L | 0.1 | 0.1 | 0.1 | 0.1 | 0.1 | 0.1 |
| NaOH | 1.0 | 1.0 | 1.0 | 1.0 | 1.0 | 1.0 |
| Water | ad 100 | ad 100 | ad 100 | ad 100 | ad 100 | ad 100 |
| pH | 8.4 | 8.4 | 8.4 | 8.4 | 8.4 | 8.4 |

| Ingredient / Formulation | 9A | 9B | 9C | 9D | 9E | 9F |
|---|---|---|---|---|---|---|
| Rhamnolipids | - | - | - | - | - | - |
| Glucolipids | 14.00 | 7.0 | 7.0 | 7.0 | 7.0 | 7.0 |
| Sophorolipids | 1.0 | 1.0 | 1.0 | 1.0 | 1.0 | 1.0 |
| C12/14 7EO (Dehydol^{®} LT7) | - | 5.0 | 5.0 | 2.5 | | 5.0 |
| SLES 2EO (Texapon^{®} N70) | - | 7.0 | 5.0 | 2.5 | 5.0 | .0 |
| Linear alkylbenzene Sulfonates (LAS) | - | - | .5 | 5 | | |
| C8C16 Alkyl polyglucosid (Glucopon^{®} 650 EC) | - | - | - | - | 2.5 | |
| Amine oxide | - | - | - | 1 | - | - |
| Sodium xylene sulfonate (Eltesol^{®} SX40) | 1.0 | 1.0 | 1.0 | 1.0 | 1.0 | 1.0 |
| Na soap from palm kernel oil | 5.00 | 5.00 | 5.00 | 5.00 | 5.00 | 5.00 |
| Amide polyglycol ether (Amidet^{®} N) | - | - | - | - | - | 1.0 |
| Polyethylene imine PEl, 20 EO (Sokalan^{®} HP20) | 2.0 | 2.0 | 2.0 | 2.0 | 2.0 | 2.0 |
| Vinylpyrrolidone/Vinylimidazole copolymer (Sokalan^{®} HP 56) | - | - | - | - | - | - |
| Texcare^{®} SRN 170 | - | - | - | - | - | - |
| Sodium citrate | 3.50 | 3.50 | 3.50 | 3.50 | 3.50 | 3.50 |
| Sodium Phosphonate (Dequest^{®} 2066) | 2.0 | 2.0 | 2.0 | 2.0 | 2.0 | 2.0 |
| Ethanol | 2.50 | 2.50 | 2.50 | 2.50 | 2.50 | 2.50 |
| MPG | 1.0 | 1.0 | 1.0 | 1.0 | 1.0 | 1.0 |
| Phenoxyethanol (Acticide^{®} SR150) | 0.45 | 0.45 | 0.45 | 0.45 | 0.45 | 0.45 |
| Liquanase^{®} 3.5 L | 1.0 | 1.0 | 1.0 | 1.0 | 1.0 | 1.0 |
| Amplify^{®} Prime 100 L | 0.4 | 0.4 | 0.4 | 0.4 | 0.4 | 0.4 |
| Lipase from example 1 | 6 U/g | 6 U/g | 6 U/g | 6 U/g | 6 U/g | 6 U/g |
| Mannaway^{®} 200 L | 0.2 | 0.2 | 0.2 | 0.2 | 0.2 | 0.2 |
| Carezyme^{®} Premium 4500 L | 0.2 | 0.2 | 0.2 | 0.2 | 0.2 | 0.2 |
| XPect^{®} 1000 L | 0.2 | 0.2 | 0.2 | 0.2 | 0.2 | 0.2 |
| Celluclean^{®} 5000 L | 0.2 | 0.2 | 0.2 | 0.2 | 0.2 | 0.2 |
| Pristine^{®} 100 L | 0.1 | 0.1 | 0.1 | 0.1 | 0.1 | 0.1 |
| NaOH | 1.0 | 1.0 | 1.0 | 1.0 | 1.0 | 1.0 |
| Water | ad 100 | ad 100 | ad 100 | ad 100 | ad 100 | ad 100 |
| pH | 8.4 | 8.4 | 8.4 | 8.4 | 8.4 | 8.4 |

| Ingredient / Formulation | 10A | 10B | 10C | 10D | 10E | 10F |
|---|---|---|---|---|---|---|
| Rhamnolipids | 14.00 | 7.0 | 7.0 | 7.0 | 7.0 | 7.0 |
| Glucolipids | - | - | - | - | - | - |
| Sophorolipids | - | - | - | - | - | - |
| C12/14 7EO (Dehydol^{®} LT7) | - | 5.0 | 5.0 | 2.5 | | 5.0 |
| SLES 2EO (Texapon^{®} N70) | - | 7.0 | 5.0 | 2.5 | 5.0 | .0 |
| Linear alkylbenzene Sulfonates (LAS) | - | - | .5 | 5 | - | - |
| C8C16 Alkyl polyglucosid (Glucopon^{®} 650 EC) | - | - | - | - | - | - |
| Amine oxide | - | - | - | - | - | - |
| Sodium xylene sulfonate (Eltesol^{®} SX40) | 1.0 | 1.0 | 1.0 | 1.0 | 1.0 | 1.0 |
| Na soap from palm kernel oil | 5.00 | 5.00 | 5.00 | 5.00 | 5.00 | 5.00 |
| Amide polyglycol ether (Amidet^{®} N) | - | - | - | - | - | - |
| Polyethylene imine PEl, 20 EO (Sokalan^{®} HP20) | - | - | - | - | - | - |
| Vinylpyrrolidone/Vinylimidazole copolymer (Sokalan^{®} HP 56) | - | - | - | - | - | - |
| Texcare^{®} SRN 170 | 1 | 1 | 1 | 1 | 1 | - |
| Sodium citrate | 6 | 6 | 6 | 6 | 6 | 6 |
| Sodium Phosphonate (Dequest^{®} 2066) | - | - | - | - | - | - |
| Ethanol | - | - | - | - | - | - |
| MPG | - | - | - | - | - | - |
| Phenoxyethanol (Acticide^{®} SR150) | 0.45 | 0.45 | 0.45 | 0.45 | 0.45 | 0.45 |
| Liquanase^{®} 3.5 L | 1.0 | 1.0 | 1.0 | 1.0 | 1.0 | 1.0 |
| Amplify^{®} Prime 100 L | 0.4 | 0.4 | 0.4 | 0.4 | 0.4 | 0.4 |
| Lipase from example 1 | 6 U/g | 6 U/g | 6 U/g | 6 U/g | 6 U/g | 6 U/g |
| Mannaway^{®} 200 L | 0.2 | 0.2 | 0.2 | 0.2 | 0.2 | 0.2 |
| Carezyme^{®} Premium 4500 L | 0.2 | 0.2 | 0.2 | 0.2 | 0.2 | 0.2 |
| XPect^{®} 1000 L | 0.2 | 0.2 | 0.2 | 0.2 | 0.2 | 0.2 |
| Celluclean^{®} 5000 L | 0.2 | 0.2 | 0.2 | 0.2 | 0.2 | 0.2 |
| Pristine^{®} 100 L | 0.1 | 0.1 | 0.1 | 0.1 | 0.1 | 0.1 |
| NaOH | 1.0 | 1.0 | 1.0 | 1.0 | 1.0 | 1.0 |
| Water | ad 100 | ad 100 | ad 100 | ad 100 | ad 100 | ad 100 |
| pH | 8.4 | 8.4 | 8.4 | 8.4 | 8.4 | 8.4 |

## Claims

1. Composition comprising
A) at least one lipase selected from Seq.-ID-No. 1 and its respective at least 60%, preferably at least 80%, more preferably at least 90%, especially preferably at least 95%, 98% or 99%, homologues at the amino acid level,
B) at least one biosurfactant, and optionally
C) at least one non-bio surfactant.

2. Composition according to Claim 1, **characterized in that** the biosurfactant is selected from the group comprising rhamnolipids, glucolipids and sophorolipids, preferably sophorolipids.

3. Composition according to Claim 1 or 2, **characterized in that** it contains component A) in an amount from 0.15 4-nitrophenyl octanoate Units / g to 2250 4-nitrophenyl octanoate Units / g, preferably from 1,5 4-nitrophenyl octanoate Units / g to 1500 4-nitrophenyl octanoate Units / g, particularly preferably from 3 4-nitrophenyl octanoate Units / g to 1050 4-nitrophenyl octanoate Units / g, based on the total composition.

4. Composition according to at least one of the preceding claims, **characterized in that** it contains component B) in an amount from 5.0 % by weight to 80% by weight, preferably from 10.0 % by weight to 75 % by weight, particularly preferably from 25 % by weight to 60 % by weight, based on the total composition.

5. Composition according to at least one of the preceding claims, **characterized in that** it contains component C) in an amount from 1.0 % by weight to 50 % by weight, preferably from 5.0 % by weight to 30 % by weight, particularly preferably from 5.0 % by weight to 20% by weight, based on the total composition.

6. Composition according to at least one of the preceding claims, **characterized in that** the non-bio surfactant is selected from the group comprising anionic, cationic, non-ionic, semi-polar and zwitterionic surfactants, more preferably anionic surfactants.

7. Composition according to at least one of the preceding claims, **characterized in that** the sum total of all surfactants is from 5.0 % by weight to 90 % by weight, preferably 20.0 % by weight to 80 % by weight, preferably 30 % by weight to 75 % by weight, wherein the percentages by weight relate to the total composition.

8. Composition according to at least one of the preceding claims, **characterized in that** the sum of all biosurfactant in the total surfactant is from 1 % by weight to 90 % by weight, preferably from 2 % by weight to 80 % by weight, particularly preferably from 51 % by weight to 60% by weight.

9. Composition according to at least one of the preceding claims comprising
D) water.

10. Composition according to at least one of the claims 1, 6 and 9 **characterized in that** it is a formulation, preferably a laundry and dishwashing formulation.

11. Composition according to Claim 10, **characterized in that** it contains component A) in an amount from 0.0015 4-nitrophenyl octanoate Units / g to 60 4-nitrophenyl octanoate Units / g, preferably from 0.015 4-nitrophenyl octanoate Units / g to 45 4-nitrophenyl octanoate Units / g, particularly preferably from 0.15 4-nitrophenyl octanoate Units / g to 15 4-nitrophenyl octanoate Units / g, based on the total composition.

12. Composition according to Claim 10 or 11, **characterized in that** it contains component B) in an amount from 0.1 % by weight to 25 % by weight, preferably from 3.0 % by weight to 20 % by weight, particularly preferably from 4.0 % by weight to 15 % by weight, based on the total composition.

13. Use of a composition according to at least one of the preceding claims to remove fat and/or oil-containing stains from a surface, preferably selected from a dish surface or a textile's surface.
